## Europäisches Patentamt
## European Patent Office
## Office européen des brevets

(11) Numéro de publication : **0 182 692**
**B1**

(12) # FASCICULE DE BREVET EUROPÉEN

(45) Date de publication du fascicule du brevet :
26.04.89

(51) Int. Cl.⁴ : **A 41 B 13/02**

(21) Numéro de dépôt : 85402085.6

(22) Date de dépôt : 29.10.85

(54) **Change à usage unique.**

(30) Priorité : 06.11.84 FR 8416843

(43) Date de publication de la demande :
28.05.86 Bulletin 86/22

(45) Mention de la délivrance du brevet :
26.04.89 Bulletin 89/17

(84) Etats contractants désignés :
AT BE CH DE FR GB IT LI LU NL SE

(56) Documents cités :
FR--A-- 2 371 893
US--A-- 3 901 237
US--A-- 3 901 237
US--A-- 3 921 638
US--A-- 3 971 380
US--A-- 4 047 528

(73) Titulaire : **KAYSERSBERG SA**
**Route de Lapoutroie**
**F-68240 Kaysersberg (FR)**

(72) Inventeur : **Ruppel, Rémy**
**7, rue des Sorbiers**
**F-68000 Horbourg (FR)**
Inventeur : **Pigneul, Raymond**
**2, rue des Vosges**
**F-68320 Durrenentzen (FR)**
Inventeur : **Tresca, Benoit**
**10, route de l'Industrie Kunheim**
**F-68320 Muntzenheim (FR)**

(74) Mandataire : **David, Daniel**
**KAYSERSBERG 54, avenue Hoche**
**F-75008 Paris (FR)**

## Description

La présente invention à pour objet un change à usage unique comportant une zone centrale d'entrejambe et deux zones de tronc situées de part et d'autre de la zone centrale, le change étant forme d'un matelas absorbant recouvert sur sa face interne d'une feuille de non-tissé et sur sa face externe d'une feuille imperméable aux liquides, les deux feuilles étant d'une aire supérieure à celle du matelas absorbant et soudées entre elles au niveau de la zone périphérique au matelas ainsi définie, une zone de tronc, du côté de chaque bord longitudinal, sur la face interne de la zone périphérique, étant pourvue d'un adhésif qui pénètre au moins partiellement la feuille perméable et recouvre la feuille imperméable.

La fixation des parties avant et arrière est assurée généralement par des attaches adhésives. Un tel article est par exemple décrit dans le brevet US-A-3971380. Bien que donnant entièrement satisfaction sur le plan technique, ces attaches adhésives augmentent sensiblement le prix de revient des articles, à cause, d'une part, du surcroît de matière que cela nécessite et d'autre part, de la complication du procédé entraînée par leur dépose sur le change.

Aussi est-il déjà connu dans l'art antérieur de supprimer de telles attaches adhésives et de les remplacer par de simples pastilles adhésives fixées de chaque côté sur la face interne de la zone de tronc arrière. Un tel dispositif est notamment décrit dans le brevet français No 2134748 du déposant. Lors de la mise en place du change les pastilles adhésives par repli des extrémités de la zone de tronc arrière sur la zone avant sont fixées sur celle-là. Un tel dispositif présente, toutefois, l'inconvénient de rendre difficile le positionnement de l'article. Ces adhésifs sont normalement protégés par un protecteur amovible. Lorsque le parent veut positionner le change, il est obligé d'enlever le protecteur avant, et court le risque que le bébé s'en saisisse pour le mettre à la bouche ou même l'avaler.

L'objet de l'invention est de remédier à cet inconvénient et, plus précisément, de fournir un change qui ne comporte plus d'attaches adhésives tout en conservant la même facilité de pose que les changes avec attaches adhésives.

L'invention est caractérisée en ce que la surface adhésivée est associée à un revêtement protecteur anti-adhérent, solidaire du change, formé par un support traité sur une face de manière à le rendre anti-adhérent, ce support présentant un retour sur la face anti-adhérente placé du côté du bord longitudinal du change, en contact avec une partie de la surface adhésivée.

En général, le change présente une forme anatomique, c'est-à-dire que les bords longitudinaux au niveau de la zone centrale sont échancrés vers l'intérieur. Cela permet une meilleure adaptation au corps du bébé. Il est également connu de placer des laminettes élastiques longitudinales dans la zone centrale de part et d'autre du matelas absorbant. Ces laminettes sont présentes dans la zone périphérique et prises en sandwich entre la feuille imperméable et la feuille perméable.

L'adhésif doit être sensible à la pression ais suffisamment fluide pour pénétrer au travers du non-tissé. En général, il s'agit d'adhésif pose a chaud (80 °C) lors de la fabrication du change. Selon un mode avantageux de réalisation, cet adhésif aura de plus une très faible résistance au pelage vis-à-vis de la feuille imperméable, ce qui permettra d'obtenir un change repositionnable. De préférence, la résistance au pelage sera comprise entre 400 et 600 C N/20 × 25 mm² et la résistance au cisaillement entre 48 et 72 N/20 x 25 mm².

Le revêtement protecteur est généralement à base de silicone et peut être constitué par le simple traitement d'une surface pour la rendre antiadhérente. Ce revêtement peut consister également en un support associé à un revêtement.

Une première mise en oeuvre consiste en ce que le revêtement protecteur antiadhérent est un support associé à la face externe de l'autre zone de tronc, sur la zone périphérique, et à proximité de chaque bord longitudinal. Ainsi, en pliant le change en trois selon un axe transversal, on obtient un article dont les adhésifs sont protégés et qui présente l'avantage supplémentaire d'être plié en trois, ce qui permet de ne pas « casser » la zone centrale du matelas.

Ce mode de réalisation présente toutefois l'inconvénient de ne pas protéger les adhésifs jusqu'au moment où ils sont posés sur la zone de tronc opposée. Aussi de préférence utilisera-t-on la solution suivante :

Elle consiste en ce que le revêtement protecteur antiadhérent est un support traité sur une face de manière à le rendre antiadhérent, ce support présentant un retour du côté du bord longitudinal du change. Ainsi, une partie de la face non traitée du support est en contact avec l'adhésif ce qui rend celui-ci fortement solidaire du change. Lorsque le support est décollé en le faisant pivoter autour de la charnière constituée par le retour il est possible de fixer la partie adhésivée sur la zone de tronc opposée, d'une part en utilisant une seule main et d'autre part, sans risquer de mettre les doigts sur l'adhésif. De préférence, le support protecteur du côté opposé au retour déborde de la partie adhésivée de manière à dégager une languette de préhension.

L'invention sera mieux comprise en se référant à un mode de réalisation particulier illustré par les dessins en annexe :

La figure 1 est une vue en plan de la face interne d'un change muni d'un adhésif et du support protecteur, un des adhésifs étant partiellement découvert.

La figure 2 est une vue en coupe transversale de la zone du change comportant l'adhésif recouvert du support protecteur.

La figure 3 est une vue en coupe transversale de la même zone que celle représentée à la figure 2 mais avec l'adhésif entièrement découvert.

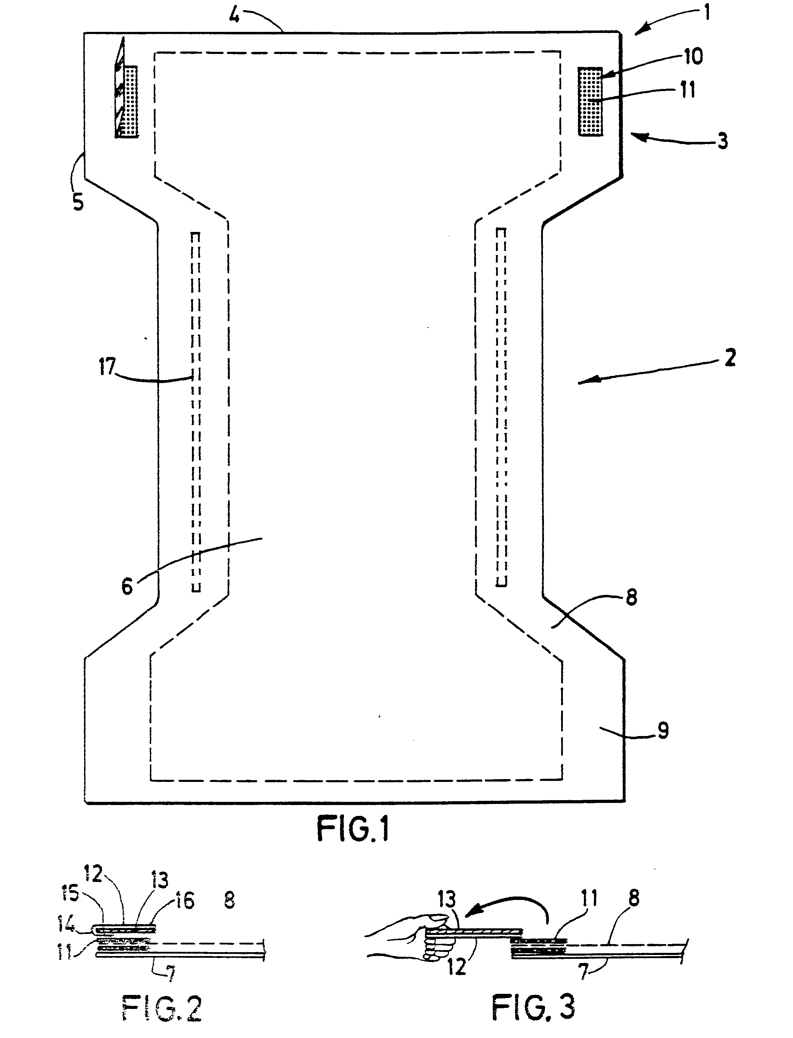

Selon les figures 1, 2, 3, le change 1 comporte dans le sens longitudinal une zone centrale 2 dite zone d'entrejambe et, de part et d'autre, une zone de tronc 3 destiné à être située lorsque le change est en position au niveau de la taille du bébé.

Le change 1 est de forme sensiblement rectangulaire limité par deux bords latéraux 4 situés sur le bébé au niveau de la taille et deux bords longitudinaux 5 entourant les cuisses. De manière connue les bords longitudinaux 5 sont incurvés vers l'intérieur au niveau de la zone d'entrejambe 2 de manière à ce que le change 1 présente une forme dite anatomique.

Le change 1 est constitué d'un matelas absorbant 6, pris en sandwich entre deux feuilles de ouate de cellulose pour améliorer sa tenue, de forme également anatomique avec des échancrures sur les bords longitudinaux. Ce matelas 6 est recouvert sur sa face externe d'une feuille imperméable 7 telle qu'une feuille de polyéthylène et sur la face interne d'une feuille de non-tissé 8 perméable aux liquides. Ces deux feuilles 7 et 8 épousent les contours du matelas 6 mais sont d'une aire égale entre elles et supérieure à celle du matelas 6. Les deux feuilles 7 et 8 sont soudées au niveau de la zone périphérique 9 ainsi obtenue où le matelas est absent. Sur la zone périphérique, au niveau d'une zone de tronc, cela peut être indifféremment la zone de tronc avant ou la zone de tronc arrière, de part et d'autre du matelas absorbant par rapport au sens longitudinal, à proximité des bords longitudinaux 5, est présente une partie 10 munie de l'adhésif 11.

L'adhésif présente les caractéristiques suivantes :

Colle thermodurcissable dont la résistance au pelage vis-à-vis d'un polyéthylène communément employé d'une épaisseur de 35μ est de 500 CN/25 x 20 mm² et la résistance au cisaillement à 0° est de 60 N/2O × 25 mm.

Cette colle est vendue par la Société française BERICOL.

L'adhésif est en outre suffisamment fluide pour que, déposé sur le non-tissé 8, il le traverse et atteint la feuille de polyéthylène. L'adhésif 11 est déposé, selon un pavé de forme rectangulaire, à proximité des bords longitudinaux du change. L'adhésif 11 est recouvert d'un support protecteur 12 traité sur la face en contact avec l'adhésif par un produit antiadhérent 13 à base de silicone par exemple. En outre, le support 12 présente d'une part sur le côté longitudinal situé à proximité du bord du change un retour 14 qui permet ainsi à une partie 15 de la face non traitée du support d'être en contact avec l'adhésif ; d'autre part, sur le côté longitudinal opposé, une extension 16 débordant de la surface adhésivée de manière à faciliter la préhension. On comprend ainsi que le retour 14 forme une charnière autour de laquelle le support protecteur 12 pivote comme le montre la figure 3. Grâce à la fixation du support au change au niveau du retour le parent peut positionner la zone de tronc arrière (ou avant) sur la zone de tronc avant (ou arrière) sans risquer de mettre les doigts sur l'adhésif. Les propriétés intrinsèques de l'adhésif rendent ce change repositionnable mais il est évident que l'invention n'est en rien limitée à cette propriété. Le change peut présenter également des laminettes élastiques 17 dans la zone centrale de part et d'autre du matelas absorbant.

## Revendications

1. Change à usage unique (1) comportant une zone centrale d'entrejambe (2) et deux zones de tronc (3) situées de part et d'autre de la zone centrale, le change à usage unique étant formé d'un matelas absorbant (6) recouvert sur sa face interne d'une feuille de non-tissé (8) et sur sa face externe d'une feuille (7) imperméable aux liquides les deux feuilles étant d'une aire supérieure à celle du matelas absorbant et soudées entre elles au niveau de la zone périphérique (9) du matelas ainsi définie, une zone de tronc, du côté de chaque bord longitudinal, sur la face interne de la zone périphérique, étant pourvue d'un adhésif (11) qui pénètre au moins partiellement la feuille perméable et recouvre la feuille imperméable, caractérisé en ce que la surface adhésivée est associée à un revêtement protecteur antiadhérent (13) solidaire du change formé par un support traité sur une face de manière à le rendre antiadhérent, ce support présentant un retour (14) sur la face antiadhérente, placé du côté du bord longitudinal du change en contact avec une partie de la surface adhésivée.

2. Change à usage unique selon la revendication 1, caractérisé en ce que sur le support protecteur du côté opposé au retour déborde de la partie adhésivée de manière à dégager une languette de préhension.

3. Change à usage unique selon l'une des revendications précédentes, caractérisé en ce que la zone adhésivée a une forme allongée dans le sens longitudinal du change.

4. Change à usage unique selon l'une des revendications précédentes, caractérisé en ce que l'adhésif possède une très faible résistance au pelage vis-à-vis de la feuille imperméable.

## Claims

1. Disposable nappy (1) comprising a central crutch zone (2) and two waist zones (3) situated on either side of the central zone, the disposable nappy consisting of an absorbant pad (6) covered on its inner side by a sheet of non-woven material (8) and on its outer side by a sheet (7) impervious to liquids, the two sheets having an area greater than that of the absorbant pad and being bonded together in the region of the peripheral zone (9) of the pad thus defined, a waist zone, in the vicinity of each longitudinal edge, on the inner side of the peripheral zone, being provided with an adhesive (11) which penetrates the permeable sheet at least partially and covers the impermeable sheet, characterized in that the adhesive surface is associated with a non-adhesive protective coating (13), integral with the nappy, consisting of a base treated on one side so as to make it non-adhesive, this base having, on the nonadhesive side, a turned-back portion (14) located in the vicinity of the longitudinal edge of the nappy and making contact with a part of the adhesive surface.

2. Disposable nappy according to Claim 1, characterized in that on (sic) the protective base on the opposite side to the turned-back portion projects from the adhesive part so as to form a gripping lug.

3. Disposable nappy according to one of the preceding claims, characterized in that the adhesive zone has an elongated shape in the longitudinal direction of the nappy.

4. Disposable nappy according to one of the preceding claims, characterized in that the adhesive has a very low peel strength with respect to the impermeable sheet.

**Patentansprüche**

1. Einmalwindel (1) mit einem zentralen Zwischenbeinbereich (2) und zwei beiderseits des zentralen Bereiches angeordneten Rumpfbereichen (3), wobei die Einmalwindel gebildet wird aus : einem absorbierenden Kissen, dessen nach innen gerichtete Fläche durch eine nicht gewebte Folie (8) und dessen nach außen gerichtete Fläche durch eine flüssigkeitsundurchlässige Folie (7) bedeckt wird, wobei die beiden Folien eine größere Fläche aufweisen als das absorbierende Kissen und miteinander auf gleicher Höhe in dem das Kissen umgebenden Bereich (9) verschweißt werden, wodurch das Kissen begrenzt wird, einem Rumpfbereich, der neben jedem Längsrand auf der nach innen gerichteten Seite der umgebenden Zone mit einem Klebstoff (11) versehen ist, der zumindest teilweise in die durchlässige Folie eindringt und die undurchlässige Folie bedeckt, dadurch gekennzeichnet, daß die klebrige Fläche mit einer Klebstoff abweisenden Schutzbedeckung (13) verbunden ist, die an der Windel befestigt ist und durch eine Einlage gebildet wird, deren eine Seite so behandelt ist, daß sie Klebstoff abweisend ist, wobei die Einlage eine Rückführung (14) auf die Klebstoff abweisende Seite aufweist, die neben dem Längsrand der Windel angeordnet ist und in Kontakt mit einem Teil der klebenden Fläche steht.

2. Einmalwindel nach Anspruch 1, dadurch gekennzeichnet, daß die Schutzeinlage auf der der Rückführung gegenüberliegenden Seite über den klebenden Teil hinausragt dergestalt, daß eine Zunge zum Ergreifen gebildet wird.

3. Einmalwindel nach einem der vorhergehenden Ansprüche, dadurch gekennzeichnet, daß die klebende Zone eine in Längsrichtung der Windel ausgerichtete, gestreckte Form aufweist.

4. Einmalwindel nach einem der vorhergehenden Ansprüche, dadurch gekennzeichnet, daß der Klebstoff einen sehr geringen Schälwiderstand gegenüber der undurchläsigen Folie aufweist.

FIG.1

FIG.2

FIG. 3